# EUROPEAN PATENT APPLICATION

(11) **EP 0 819 769 A2**
(43) Date of publication of application: **21.01.1998**
(21) Application number: 97112232.0
(22) Date of filing: 17.07.1997
(51) Int. Cl.: C12Q 1/68

(54) **A method of flow cytometric determination of telomerase activity**

(30) Priority: 17.07.1996 JP 187350/96
(71) Applicant: KYOWA HAKKO KOGYO CO., LTD., Chiyoda-ku, Tokyo-to (JP)
(72) Inventor: Ohyashiki, Kazuma, Setagaya-ku, Tokyo-to (JP); Ohyashiki, Junko, Setagaya-ku, Tokyo-to (JP)
(74) Representative: VOSSIUS & PARTNER

(57) **Abstract**

There are provided a quantitative and simple method of determining telomerase activity and a method of detecting a telomerase positive cell, said methods comprising:
(i) a step of conducting elongation reaction of telomerase substrate with telomerase in cells;
(ii) a step of subjecting the resulting elongation reaction product to base binding chain reaction in cells; and
(iii) a step of quantitatively determining fluorescent light derived from the reaction product in cells using flow cytometry.

## Description

The present invention relates to a quantitative and simple method of determining the activity of telomerase which plays an important role in division and proliferation of cancer cells and which is an important approach to be determined in a clinical laboratory test. The present invention also relates to a method of detecting a telomerase positive cell. The present invention is significant in clinical laboratory testing technology, for example, for diagnosing cancer and observing its progress.

The methods of determining telomerase activity is proceeded by the following steps. A radioisotope labeled primer is subjected to telomerase reaction, and, after amplifying the telomerase reaction product by polymerase chain reaction (PCR), the PCR reaction product is subjected to electrophoresis followed by autoradiography, thereby determining the activity (Kim, et al., Science, 266, 2011 (1994); WO 95/13381).

Since the sample for the methods of the prior art is restricted to the cell extract etc., the methods of the prior art can not detect individual cellular telomerase activity. Therefore, the methods of the prior art can not be applied to a diagnosis at a cellular level, employed frequently in diagnosing cancers.

Accordingly, a main object of the present invention is to provide a simple method in determining telomerase activity which can quantitatively determine telomerase activity at the cellular level and which is applicable to a general test laboratory for the purpose of early detection of cancers.

This object as well as other objects and advantages of the present invention will become apparent to those skilled in the art from the following description.

In order to solve the problems mentioned above, the following unique method of determining telomerase activity in cells is provided. (1) The telomerase reaction is directly conducted in cells using a fluorescent-labeled substrate, (2) then a base binding chain reaction (PCR reaction) is conducted using the obtained fluorescent-labeled primer, and (3) then the generated intracellular telomerase reaction-induced fluorescent light is quantitatively and conveniently identified by flow cytometry. Reagents which are used for the unique method are also provided. Further, there are provided a method of detecting a telomerase positive cell by analyizing the histogram from the flow cytometry obtained during the step of the above determining method, as well as a reagent for detecting the same.

The present invention relates to a method of determining telomerase activity comprising:
(i) a step of conducting elongation reaction of telomerase substrate with telomerase in cells;
(ii) a step of subjecting the resulting elongation reaction product to base binding chain reaction in cells; and
(iii) a step of quantitatively determining fluorescent light derived from the reaction product in cells using flow cytometry.

The present invention also relates to a method of detecting a telomerase positive cell comprising:
(i) a step of conducting elongation reaction of telomerase substrate with telomerase in cells;
(ii) a step of subjecting the resulting elongation reaction product to base binding chain reaction in cells; and
(iii) a step of quantitatively determining fluorescent light derived from the reaction product in cells using flow cytometry.

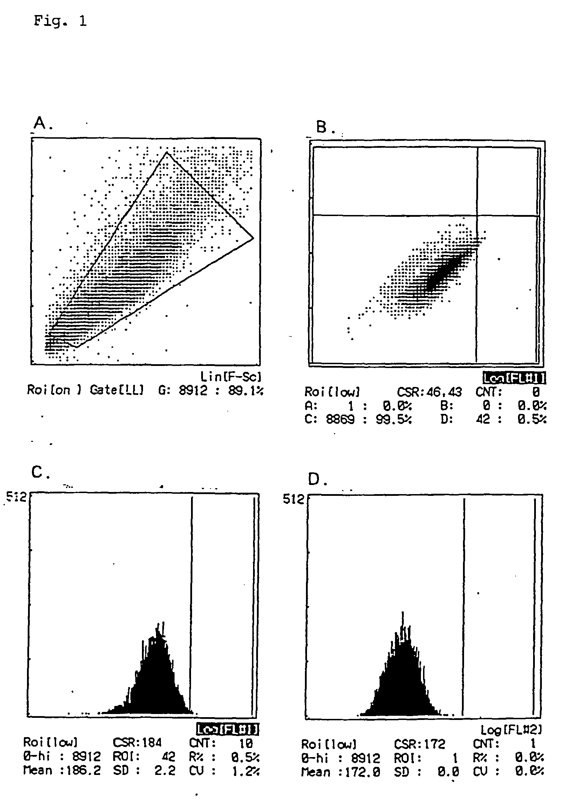

Fig.1 is a view showing fluorescent intensity in K526 cells after PCR. Fig. 1A is a view showing the relationship between the size per 10,000 cells (ordinate) and the fluorescent intensity (abscissa). Fig. 1B is a view showing the relationship between the cell size (ordinate) and the fluorescent intensity (abscissa) after gating A. Fig. 1C is a view showing the relationship between the number of cells (ordinate) and the fluorescent intensity (abscissa). Fig. 1D is a view showing the relationship between the number of cells (ordinate) and the cell size (abscissa).

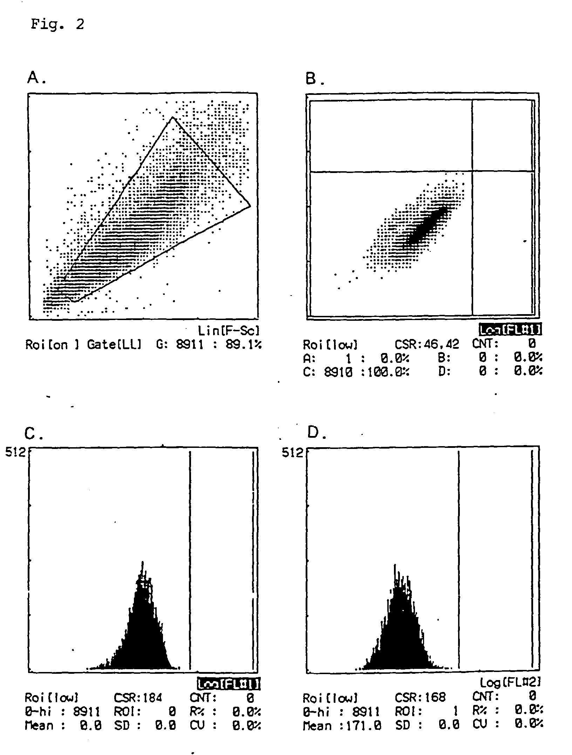

Fig. 2 is a view showing fluorescent intensity in K562 cells not treated with PCR. Fig. 2A is a view showing the relationship between the size per 10,000 cells (ordinate) and the fluorescent intensity (abscissa). Fig. 2B is a view showing the relationship between the cell size (ordinate) and the fluorescent intensity (abscissa) after gating A. Fig. 2C is a view showing the relationship between the number of cells (ordinate) and the fluorescent intensity (abscissa). Fig. 2D is a view showing the relationship between the number of cells (ordinate) and the cell size (abscissa).

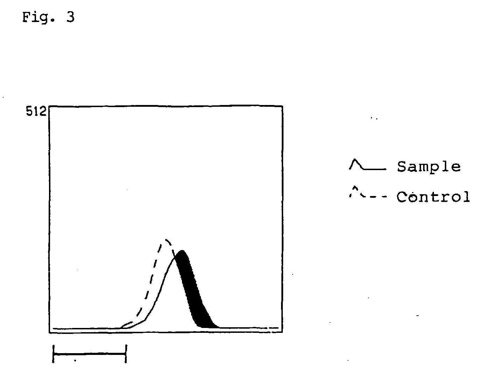

Fig. 3 is a view showing the change in fluorescent intensity in K562 cells between before and after PCR.

The step of conducting elongation reaction of telomerase substrate with telomerase in cells is a step wherein a fluorescent-labeled telomerase substrate and deoxynucleoside triphosphates are added to a cell as a sample and wherein elongation reaction of telomerase substrate is conducted in the cell.

The cell as a sample may be any cell which can be separated by flow cytometry, such as blood cell, lymphoid cell, cultured cell, suspending cell and the like.

The cells taken from viable organisms and the like may be suspended in a culture medium for cultivating cells or in 1 to 300 mM phosphate buffer, pH 6.5 to 8.5, or the like, or may be placed to stand on a microtube container and, while maintaining the cells at these states, they may be used for substrate elongation reaction. After collecting the cells, they are stored at a temperature of not higher than 15 °C, preferably below or at 4 °C, and are then preferably used for substrate elongation reaction immediately after storage. The culture medium for cultivating the cells may be any medium which is normally used for cultivating suspended cells, wherein RPMI 1640, modified Dulbecco's mineral essential medium and the like are preferable.

The telomerase according to the present invention (EC 2.7.7.31) means an enzyme involved in synthesis of repeating sequence of telomere (telomere repeating sequence) which is the terminal structure essential for a linear chromosome having self-repair functions. The telomere means a terminal moiety of a straight chain chromosome and the telomere repeating sequence means a repeating sequence of a certain base sequence localized in the telomere.

The telomerase substrate is preferably an oligonucleotide containing no telomeric sequence and may be 5'-GTTAGGGTTAGGGTTAGG-3', 5'-TTAGGGTTAGGGTTAGGG-3' and the like, although the most preferable substrate is TS primer (5'-AATCCGTCGAGCAGAGTT-3').

The fluorescent-labeled form of the telomerase substrate means a telomerase substrate containing a telomerase substrate bound to a fluorescent label. The telomerase substrate bound to the fluorescent label can be obtained by binding a fluorescent reagent such as fluorescent phosphoramidite (Manufactured by CLONETECH) to the telomerase substrate (Makino, et al., PCR methods and applications (1992), Published by COLD SPRING HARBOR LABORATORIES).

The deoxynucleoside triphosphates mean the mixtures of deoxynucleoside triphosphates and consist of deoxyadenosine triphosphate, deoxyguanosine triphosphate, deoxycytidine triphosphate and deoxythymidine triphosphate.

The telomerase substrate elongation reaction means a reaction by which a telomere repeating sequence originating from the fluorescent substance of the telomerase substrate is synthesized by means of the telomerase contained in the cells. More particularly, various deoxynucleoside triphosphates such as deoxyadenosine triphosphate are bound to the fluorescent substance of the telomerase substrate according to the telomere repeating sequence by means of the telomerase contained in the sample, resulting in the synthesis of the elongated form of the telomerase substrate.

The telomerase substrate elongation reaction may be conducted by a method described in the literature by LITE (LITE, et al., NUCLEOSIDES AND RESEARCH 23, 3794 (1955)). After adding a fluorescent-labeled form of the telomerase substrate and a buffer solution containing deoxynucleoside triphosphates to the cells, the sample is incubated.

In this step, when the cells are suspended, it is preferable to initiate the reaction by adding the buffer solution to the cells which have been sedimented to the bottom of the microtube or the like after centrifugation. In this step, it is preferable to supplement the buffer solution with either a heat-resistant (Taq) base binding enzyme for the base binding enzymatic chain reaction or a primer. The incubation is conducted preferably in a chamber protected from light.

The buffer solution may be any of those at a concentration of 1 to 100 mM and at a pH of 6 to 9, preferably 7.5 to 8.5, although phosphate buffers and Tris-HCl buffers are preferred. Such buffer solutions may further contain inorganic salts such as potassium chloride, calcium chloride, sodium chloride and magnesium chloride, surfactants such as polyoxyethylene sorbitan monolaurate (hereinafter referred to as Tween 20), chelating agents such as ethylene glycol bis(2-aminoethylethyl)-N,N,N',N'-tetraacetic acid (hereinafter referred to as EGTA) as well as proteins such as T4 gene 32 protein and bovine serum albumin.

In cases where PCR is employed for the base binding enzymatic chain reaction, the primer may be any of those constructed from incomplete telomeric repeating sequences (5'-CCCTTA-3') and complete telomeric repeating sequences (5'-CCCTAA-3'), and it is preferable to employ a fluorescent-labeled CX reverse primer constructed from three incomplete telomeric repeating sequences and one complete telomeric repeating sequence (5'-CCCTTACCCTTACCCTTACCCTAA-3'). In cases where ligase chain reaction (LCR) is employed for the base binding enzymatic chain reaction, any of those constructed from C-rich telomeric sequences (5'-CTAACC-3') may be employed, and it is preferable to employ a fluorescent-labeled ACT primer (5'-GCGCGGCTAACCCTAACCCTAACC-3').

Such primers as well as the following oligonucleotide primers described in the present specification can be obtained by a standard manner using an automatic DNA synthesizer.

The fluorescent-labeled CX reverse primer or fluorescent-labeled ACT primer means a CX reverse primer or ACT primer bound to a fluorescent label, respectively. The CX reverse primer or ACT primer bound to a fluorescent label can be obtained by binding a fluorescent reagent such as fluorescent phosphoramidite (Manufactured by CLONETECH) to the CX reverse primer or ACT primer (Makino, et al., PCR method and applications (1992), Published by COLD SPRING HARBOR LABORATORIES).

The heat-resistant (Taq) base binding enzyme represents a Taq DNA polymerase (E.C.2.7.7.7) when employing PCR in the base binding enzymatic chain reaction and a Taq DNA ligase (E.C.6.5.1.1) when employing LCR.

The base binding enzymatic chain reaction may be PCR and LCR, based on the selection from which the heat resistant base binding enzyme and the primer are determined as mentioned above.

More particularly, the telomerase substrate elongation reaction product and the primer may be subjected to the PCR reaction to amplify using the Taq DNA polymerase (SAIKI, et al., Science 239, 487 (1988); Kim, et al., Science 266, 2011 (1994)), or , alternatively, the telomerase substrate elongation reaction product and the primer may be subjected to the LCR reaction to amplify using the Tag DNA ligase (BALANEY, et al., Proc. Natl. Acad. Sci., 87 1874 (1987)).

By combining the heat resistant base binding enzyme and the primer appropriately as described above, the base binding enzymatic chain reaction is initiated, and, in order to conduct this reaction, it is preferable to employ, for example, a commercially available automatic PCR system (Tradename: THERMALCYCLER, manufactured by Perkin Elmer).

After completion of the base binding enzymatic chain reaction, the cells obtained by centrifugation are washed with the above buffer solution. The resulting cells are suspended for use in flow cytometry according to a conventional method (Hositani, et al., Protein, Nucleic Acid, Enzyme, 39, 1888-1898, 1994). The resulting sample is injected into a fluorocytometer and, thereby, the fluorescent intensity in the cells can be detected with fluorocytometry. In this step, the total fluorescent intensity of the resulting sample can be obtained and, thereby, the telomerase activity in the sample can be quantitatively determined. Alternatively, in cases where the cells in the sample are separated using a cell sorter, the telomerase activity in each population of the cells can be quantitatively determined.

Further, detection of a telomerase positive cell and identification of the ratio of positive cells relative to total cells can be conducted by making a histogram using features such as cell size in the sample, fluorescent intensity and the like.

Detection using flow cytometry represents as follows: for example, a fluorescent-labeled base binding enzymatic chain reaction product which is present in the cells and has been amplified by PCR is irradiated with laser light having excitation wavelength of 488 nm, therby measuring five kinds of parameters at once, every time when one cell passes therethrough, using five photodetectors and, thereby the fluorescent-labeled cells are detected. The five kinds of parameters are forward scattered light (FSC: forward scatter), side scattered light (SSC: side scatter) and three kinds of fluorescent lights (FL: fluorescence). The fluorescent-labeled primer which has been amplified by the reaction is detected in a vertical direction relative to laser light (Detection of lymphocyte functions, Ed. by J. Yada and M. Fujiwara., Published by Chugaiigaku, 15-53 (1994))). In order to detect the reaction, a commercially available flow cytometer or the like is preferably used [Medical Technology, 22, 965-974 (1994)]. Further, the conditions such as laser light wavelength and the like in flow cytometry may vary depending upon the kind of the fluorescent reagent for labeling the primer.

The present invention is further described in detail below.

From a sample obtained from a viable organism, cells are harvested in a standard manner using, for example, centrifugation. The sample may be cells which are separated after having been finely excised from tissues. After washing the cells harvested in a cell culture medium (RPMI 1640 and the like) or phosphate buffer solution or the like, the cells are recovered into a microtube which is centrifuged to remove the supernatant.

1 to 100 mM Tris-HCl buffer (pH 6 to 9) is supplemented with 1 to 1000 pmol, preferably 5 to 200 pmol, more preferably 10 pmol fluorescent-labeled TS primer (5'-AATCCGTCGAGCAGAGTT-3'), 1 to 300 mM deoxynucleoside triphosphates and 0.1 to 20 units of Taq DNA polymerase and added to the sample in the microtube to suspend the cells. The suspension is incubated at a temperature of 10 to 50 °C, preferably 20 to 40 °C, more preferably at 25 °C, for a period of 10 minutes to 1 hour, preferably for 30 minutes, in a chamber protected from light.

After completion of the TS primer elongation reaction, 1 to 300 pmol, preferably 50 to 200 pmol, more preferably 10 pmol fluorescent-labeled CX reverse primer (5'-CCCTTACCCTTACCCTTACCCTAA-3') is added, thereby initiating PCR, in which a thermal reaction cycle of PCR may be, for example, 1 to 120 seconds at 90 to 100 °C, 1 to 120 seconds at 40 to 60 °C, and 1 to 240 seconds at 60 to 80 °C, which are repeated 1 to 50 times. In order to conduct PCR, the THERMALCYCLER mentioned above or equivalent is preferably employed.

After completion of PCR and washing the cells with the phosphate buffer solution, the cells are resuspended in the phosphate buffer solution, and a flow cytometer can be used to quantitatively determine the telomerase activity in the sample, to quantitatively determine the telomerase activity in each population of cells sorted by a cell sorter, and to identify the ratio of telomerase positive cells in the sample.

Identification of the ratio of positive cells using flow cytometry is calculated as follows.
(1) The positive is defined as fluorescent development in cells treated after all step reactions.
(2) The negative is defined as fluorescent development in cells treated after all step reactions except for PCR reaction.
(3) The telomerase positive cell ratio is defined as the value subtracting the negative from the positive, because the value depends on the enhanced fluorescence of the cells.

The telomerase activity assay reagent and the telomerase positive cell assay reagent are also provided by combining any of the compounds such as telomerase substrate, primer, deoxynucleoside triphosphate, Taq DNA polymerase, T4 gene 32 protein and the like, the buffer solutions mentioned above, washing fluids or the various additives thereto as listed above, which are used in the present invention, and devices such as microtubes and the like, which are used in the present invention.

The following examples further illustrate the present invention in detail but are not to be construed to limit the scope thereof.

### Example 1

Leukemia cell line K562 cells were harvested. The cells harvested were washed with RPMI 1640 culture medium at 4 °C, then recovered using a centrifuge, and transferred into a microtube.

25 µl of 20 mM Tris-HCl buffer (pH 6 to 9) [containing 1.5 mM magnesium chloride, 63 mM potassium chloride, 0.05% Tween 20 (Tradename), 1 µM EGTA, 1 µg/ml T4 gene 32 protein (Manufactured by Boehringer Mannheim), bovine serum albumin (0.1 mg/ml)] was supplemented with 10 pmol fluorescent-labeled TS primer (5'-AATCCGTCGAGCAGAGTT-3') [Manufactured by PHARMACIA BIOTECH], 50 mM deoxynucleoside triphosphates and 2 units of a Taq DNA polymerase and added to the sample in the microtube. The microtube was incubated at 22 °C for 30 minutes in a chamber protected from light.

After completion of the TS primer elongation reaction, the microtube was treated at 90 °C for 1.5 minutes in order to inactivate the telomerase. Thereafter, 25 µl of 10 pmol fluorescent-labeled CX reverse primer (5'-CCCTTACCCTTACCCTTACCCTAA-3') was added to the sample in the microtube. Upon this addition, PCR was initiated and PCR was conducted in a THERMALCYCLER mentioned above. The thermal cycles were 30 seconds at 94 °C, 30 seconds at 50 °C and 90 seconds at 72 °C, which were repeated 30 times.

After completion of PCR, the sample in the microtube was washed twice with the phosphate buffer solution (pH 6.4) and suspended in 100 µl of phosphate buffer. The fluorescent-labeled cells were subjected to flow cytometry to identify the ratio of telomerase positive cells in the sample. For flow cytometry examination, 10,000 cells which had been subjected to PCR were used and the conditions of flow cytometry were set so that the fluorescent intensity was indicated in the abscissa and the cell size was indicated in the ordinate (Fig. 1A).

Cell size and fluorescent intensity measured in flow cytometry were gated (in frame in Fig. 1A), a computer provided on flow cytometry was used to integrate fluorescent intensity of each cell and the computer was set so as to indicate the fluorescent intensity in the abscissa and the number of cells in the ordinate (Fig. 1). Similarly, fluorescent intensity and number of cells were measured in cells treated similarly but eliminating only PCR treatment (Fig. 2). Detection of telomerase positive cells was conducted by obtaining the difference between cells treated with PCR (Fig. 1) and cells treated similarly but eliminating only PCR treatment (Fig. 2) (Fig. 3), which difference was adopted as enhanced rate derived from cells which exhibited enhanced fluorescence and, thus, this value was adopted as telomerase positive cell rate.

In order to confirm whether a fluorescent signal, derived from K562 cells, which had been detected in flow cytometry, depends upon telomerase activity or not, the same sample after elongation reaction and PCR using a fluorescent-labeled primer was immobilized on a slide glass, and the sample was observed by a fluorescent microscope. Observation by a fluorescent microscope represents a morphological observation using a G-filter in order to identify whether fluorescent-labeled cells were ruptured by the reaction or not. Since the sample omitting only PCR treatment (Fig. 2) and a sample eliminating only the addition of TS primer exhibited attenuated fluorescence, it is suggested that the fluorescent peaks were attributable to the PCR products synthesized from the intracellular telomerase products.

If a difference between the positive (fluorescent intensity in cells treated after all step reactions) and the negative (fluorescent intensity in cells omitting only PCR treatment) (Fig. 3) was observed, then the cells with enhanced fluorescence were evaluated as positive cells by computer. The positive ratio was found to be 39.3%.

### Example 2

According to a similar manner, telomerase positive cells from various cell resources were calculated using flow cytometry. The results are shown in Table 1.

**Table 1**

| Cell resources | Telomerase positive cells |
|---|---|
| Normal human peripheral blood mononuclear cell | 4.29% |
| PHA-stimulated normal human mononuclear cell | 64.52% |

| Leukemia cell strain | |
|---|---|
| HEL | 67.02% |
| ML-1 | 65.18% |
| U937 | 54.74% |
| HAL-01 | 65.63% |
| TALL2 | 27.00% |
| HL60 | 97.19% |

Concerning above cells in Table 1, it was known that telomerase activity is as low as 0.8 in normal human mononuclear cells while high telomerase activity is found in leukemia cell strains (K. Oyashiki., et al., International Journal of Oncology, 8, 417 (1996)). Since the telomerase positive cells in Table 1 show the same tendency, it was found that enhanced fluorescence by PCR is derived from telomerase activity.

Based on the results described above, it was shown that telomerase activity in cells can be determined quantitatively and conveniently and the ratio of telomerase activity positive cells can be determined by the intracellular telomerase assay method using flow cytometry according to the present invention.

As described above, according to the present invention, an assay method and an assay reagent useful in determining telomerase positive cells which are essential for clinical diagnosis are provided. Since the method according to the invention is a convenient method of determining the ratio of telomerase positive cells using flow cytometry and is applicable to the diagnosis of cancer cells which is conducted frequently in the field of clinical medicine, it can be utilized in clinical laboratory tests, pathological tests and the like.

## Claims

1. A method of determining telomerase activity comprising:
(i) a step of conducting elongation reaction of telomerase substrate with telomerase in cells;
(ii) a step of subjecting the resulting elongation reaction product to base binding chain reaction in cells; and
(iii) a step of quantitatively determining fluorescent light derived from said reaction product in cells using flow cytometry.

2. The method according to claim 1, wherein the step of conducting elongation reaction of telomerase substrate with telomerase in cells comprises the step of conducting elongation reaction of telomerase substrate with telomerase in cells by adding a fluorescent-labeled form of the telomerase substrate and deoxynucleoside triphosphates to cells employed as the sample.

3. The method according to claim 1 or 2, wherein the base binding chain reaction is PCR reaction or LCR reaction.

4. A method of detecting a telomerase positive cell comprising:
(i) a step of conducting elongation reaction of telomerase substrate with telomerase in cells;
(ii) a step of subjecting the resulting elongation reaction product to base binding chain reaction in cells; and
(iii) a step of quantitatively determining fluorescent light derived from said reactions product in cells using flow cytometry.

5. The method according to claim 4, wherein the step of conducting elongation reaction of telomerase substrate with telomerase in cells comprises the step of conducting elongation reaction of telomerase substrate with telomerase in cells by adding a fluorescent-labeled form of the telomerase substrate and deoxynucleoside triphosphates to cells employed as the sample.

6. A method of determining telomerase activity according to claim 4 or 5, wherein base binding chain reaction is PCR reaction or LCR reaction.

7. A telomerase activity assay kit which comprises a telomerase substrate, a primer, deoxynucleoside triphosphate, Taq DNA polymerase, and T4 gene 32 protein.

8. A method of identifying the ratio of telomerase positive cells in a sample comprising
steps (i) to (iii) as defined in claim 1 for defining the positive;
steps (i) and (iii) as defined in claim 1 for defining the negative; and
substracting the negative from the Positive to result in the ratio of telomerase positive cells whereby a difference between the positive and the negative indicates telomerase positive cells.


SEQUENCE LISTING

(1) GENERAL INFORMATION:

(i) APPLICANT:
(A) NAME: Kyowa Hakko Kogyo Co., Ltd.
(B) STREET: 6-1, Ohtemachi 1-chome
(C) CITY: Chiyoda-ku, Tokyo-to
(D) STATE: Tokyo
(E) COUNTRY: Japan
(F) POSTAL CODE (ZIP): none

(ii) TITLE OF INVENTION: A method of flow cytometric determination of
telomerase activity

(iii) NUMBER OF SEQUENCES: 5

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30 (EPO)

(v) CURRENT APPLICATION DATA:
APPLICATION NUMBER: EP 97 11 2232.0-2116

(2) INFORMATION FOR SEQ ID NO: 1:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "oligonucleotide"

(iii) HYPOTHETICAL: YES

(xi) SEQUENCE DESCRIPTION: SEQ ID NO: 1:

GTTAGGGTTA GGGTTAGG 18

(2) INFORMATION FOR SEQ ID NO: 2:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "oligonucleotide"

(iii) HYPOTHETICAL: YES

(xi) SEQUENCE DESCRIPTION: SEQ ID NO: 2:

TTAGGGTTAG GGTTAGGG 18

(2) INFORMATION FOR SEQ ID NO: 3:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "oligonucleotide"

(iii) HYPOTHETICAL: YES

(xi) SEQUENCE DESCRIPTION: SEQ ID NO: 3:

AATCCGTCGA GCAGAGTT 18

(2) INFORMATION FOR SEQ ID NO: 4:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "oligonucleotide"

(iii) HYPOTHETICAL: YES

(xi) SEQUENCE DESCRIPTION: SEQ ID NO: 4:

CCCTTACCCT TACCCTTACC CTAA 24

(2) INFORMATION FOR SEQ ID NO: 5:

(i) SEQUENCE CHARACTERISTICS:
(A) LENGTH: 24 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "oligonucleotide"

(iii) HYPOTHETICAL: YES

(xi) SEQUENCE DESCRIPTION: SEQ ID NO: 5:

GCGCGGCTAA CCCTAACCCT AACC 24